# EUROPÄISCHE PATENTSCHRIFT

(11) **EP 0 657 254 B1**
(45) Veröffentlichungstag und Bekanntmachung des Hinweises auf die Patenterteilung: **03.12.1997**
(21) Anmeldenummer: 94117058.1
(22) Anmeldetag: 28.10.1994
(51) Int. Cl.: B25F 1/00, A61C 13/12, A61C 7/02, B25B 7/22, B25B 7/02

(54) **Kombinationswerkzeug**
Combination tool
Outil combiné

(30) Priorität: 10.11.1993 DE 4338292; 03.09.1994 DE 9414329 U
(43) Veröffentlichungstag der Anmeldung: 14.06.1995
(73) Patentinhaber: ORBIS-Werk Groten GmbH + Co. KG, 48683 Ahaus (DE)
(72) Erfinder: Kalthoff, Ferdinand, D-48366 Laer (DE)
(74) Vertreter: Habbel, Hans-Georg, Dipl.-Ing.

(56) Entgegenhaltungen:
- WO-A-91/11296
- DE-U- 8 902 490
- DE-U- 9 108 207
- FR-A- 930 571
- FR-A- 975 838
- FR-A- 1 010 921
- GB-A- L21 523
- GB-A- 577 653
- US-A- 4 050 152
- DATABASE WPI Week 9150, Derwent Publications Ltd., London, GB; AN 91-368115 & US-A-5 067 240 ((TOUY/) YOU Y J)

## Beschreibung

Die Erfindung betrifft ein Kombinationswerkzeug nach dem Oberbegriff des Anspruches 1.

Ein derartiges Kombinationswerkzeug ist aus der DE-A-24 21 738 bekannt.

Das bekannte Werkzeug soll ausschließlich auf der Ausbildung als Kombizange basieren, bei der wie auch aus der Zeichnung dieser bekannten Druckschrift ersichtlich ist, die beiden Backen der Zange eine relativ großflächige, teilweise gezahnte Kontaktfläche miteinander aufweisen. Auf diese Weise ist eine gute Übertragung der Schlagenergie möglich.

Bei dem bekannten Kombinationswerkzeug ist nachteilig, daß es für einige spezielle Anwendungsfälle, beispielsweise im Bereich der Dentaltechnik, nicht einsetzbar ist, da dort Kombizangen nicht oder nicht vorteilhaft einsetzbar sind.

Der Erfindung liegt die Aufgabe zugrunde, ein gattungsgemäßes Kombinationswerkzeug dahingehend zu verbessern, daß es im Bereich der Dentaltechnik Verwendung finden kann.

Diese der Erfindung zugrundeliegende Aufgabe wird durch die Ausgestaltung gemäß dem kennzeichnenden Teil des Anspruches 1 gelöst.

Überraschend hat sich gezeigt, daß bei Ausgestaltung der beiden Wirkteile in Form einer Gipszange auch ohne eine große Kontaktfläche der beiden Backen miteinander, die Übertragung einer ausreichenden Schlagenergie möglich ist, um die in der Dentaltechnik üblichen Werkstoffe zu bearbeiten, beispielsweise Gips.

Dabei ermöglicht das erfindungsgemäße Kombinationswerkzeug eine erhebliche Arbeitsersparnis:

Für den Bereich der Gaumen- und Zahnabformungen werden Gipsmodelle verwendet. Diese Gipsmodelle müssen aus einem metallischen Formkasten ausgebettet werden, wobei üblicherweise ein Hammer dazu dient, die oberen und unteren Deckelplatten der Metallform abzuschlagen, um diese öffnen zu können.

Sobald der metallische Formkasten mit Hilfe des Hammerkopfes so weit gelockert worden ist, daß ein Spalt entstanden ist, wird üblicherweise die Hammerspitze in diesen Spalt gesteckt und durch ein Verdrehen des Hammers der Spalt so weit geöffnet, daß die Deckelplatten entnommen und die Form geöffnet werden kann.

Weiterhin wird die Sockelplatte bestimmter Gipsmodelle, die beispielsweise aus Kunststoff bestehen kann, mit Hilfe des Hammers vom eigentlichen Gipsmodell getrennt. Hierzu wird üblicherweise ein Gipsmesser an den Grenzbereich zwischen Sockelplatte und Modell angesetzt und dann mit dem Hammer auf den Messerrücken eingeschlagen. Nachteilig ist hierbei, daß es häufig zu Verformungen des Gipsmessers kommt, so daß dessen Lebensdauer erheblich verkürzt wird.

Weiterhin wird bei einigen Gipsmodellen ein angeformter Gipsrand für das Modell hergestellt, der anschließend entfernt, nämlich abgebrochen oder abgeschnitten werden muß. Hierzu wird üblicherweise eine Gipsschere verwendet.

Insgesamt ist daher bei der Bearbeitung eines Gipsmodells von der Handhabung des zunächst noch geschlossenen metallischen Formkastens bis zur Bearbeitung des Gipsmodell selbst die Handhabung einer Vielzahl unterschiedlicher Werkzeuge erforderlich. Dabei sind manche Werkzeuge für verschiedene Bearbeitungsschritte zu gebrauchen, so daß ein ständiger Wechsel der Werkzeuge erforderlich ist.

Durch das erfindungsgemäße Werkzeug wird ermöglicht, daß sowohl der metallische Formkasten geöffnet, als auch anschließend das Gipsmodell bearbeitet werden kann, ohne daß hierzu unterschiedliche Werkzeuge gehandhabt werden müssen. Hierdurch wird eine Erleichterung und Beschleunigung der Arbeit an den Gipsmodellen ermöglicht.

Eine gute Handhabung des aus nunmehr Hammerkopf und Hammerspitze zweiteilig aufgebauten Hammers wird dadurch ermöglicht, daß diese beiden Bestandteile des Hammers im wesentlichen senkrecht zur Längsachse der Zange verlaufen, ähnlich wie Hammerkopf und Hammerspitze im wesentlichen senkrecht zum Griff eines handelsüblichen Hammers angeordnet sind.

Weiterhin wird eine achsgleiche Ausrichtung von Hammerkopf und Hammerspitze vorgesehen, so daß beispielsweise beim Auftreffen der Hammerspitze das Gewicht des Hammerkopfes die Schlagwirkung noch unterstützt und nicht durch einen axialen Versatz zu einem Verreißen der Hammerspitze führt.

Vorteilhaft wird an der Hammerspitze eine besonders schmale vordere Kante angeformt, die gegenüber den handelsüblichen Hämmern schmaler ausgebildet sein kann. Hierdurch wird ermöglicht, beispielsweise beim Abschlagen von Sockelplatten unmittelbar mit der Hammerspitze auf die Trennfuge einzuwirken. Die Benutzung eines Gipsmessers erübrigt sich daher, so daß dessen Lebensdauer einerseits verlängert wird und andererseits ein erneuter Werkzeugwechsel überflüssig wird.

Die Schneidkanten der Gipsschere können in an sich bekannter Weise Vertiefungen aufweisen, um einen Verzahnungseffekt zwischen der Schneidkante und dem Gipsmodell zu erreichen, wenn mit Hilfe der Gipsschere Teilbereiche des Gipsmodells abgebrochen werden sollen. Vorteilhaft können hierbei die einzelnen Zähne mit Hilfe entsprechend angebrachter Vertiefungen so ausgebildet werden, daß sie im wesentlichen senkrecht zur Schneidkante bzw. senkrecht zur Längsachse des Kombinations-Dentalwerkzeuges angeordnet sind, während bei bekannten Gipsscheren die Verzahnungen gegenüber der Scherenspitze widerhakenartig nach hinten gerichtet sind.

Hierdurch wird ein Abreißen von Gipsstücken am Modell begünstigt, wobei häufig unerwünscht auch Bereiche des Gipsmodells abgerissen werden, die an sich am Modell verbleiben sollen. Durch die erfindungsgemäße Anordnung der Vertiefungen wird ein gerader Schnitt durch das Gipsmaterial begünstigt, so daß definierte und sauber begrenzte Bereiche mit Hilfe der erfindungsgemäß ausgebildeten Gipsschere entfernt werden können und die Gefahr reduziert wird, daß unerwünschte Beschädigungen am Modell erfolgen. Die Vertiefungen können in den Schneidflächen als Nuten ausgebildet sein, die sich bis zur Schneidkante erstrecken. Sie können jedoch auch als regelrechte Verzahnungen in der Schneidkante selbst eingeformt sein, so daß die innere Kontur einer mit derartigen Vertiefungen versehenen Backe gezahnt ausgebildet ist.

Zwischen den beiden Griffteilen kann eine Feder vorgesehen sein, die die beiden Griffteile spreizt, um auf diese Weise die Handhabung des Kombinationswerkzeuges zu erleichtern und insbesondere ein einhändiges Arbeiten zu erleichtern, bei dem der ständige Wechsel von Schließen und Öffnen der Zange durch eine derartige Feder erleichtert wird.

Vorteilhaft wird dabei die Feder oberhalb des eigentlichen Griffbereiches der beiden Griffteile angeordnet, also zwischen diesem Griffbereich und dem Gelenk des Kombinationswerkzeuges. Auf diese Weise ist jede Verletzungs- oder Klemmgefahr ausgeschlossen, da sich die Feder außerhalb des Bereiches befindet, in dem der Benutzer das Werkzeug anfaßt und handhabt.

Weiterhin kann vorteilhaft für die Benutzung des Werkzeuges als Hammer ein Fanghaken vorgesehen sein, mit dessen Hilfe das Werkzeug in seinem geschlossenen Zustand gehalten werden kann. Dabei kann vorteilhaft auch dieser Fanghaken zwischen dem eigentlichen Griffbereich der Griffteile und dem Gelenk angeordnet sein, um Behinderungen oder gar Verletzungen des Benutzers auszuschließen.

Um störende Einflüsse des Fanghakens auszuschalten, die durch einen frei beweglichen Fanghaken hervorgerufen werden könnten, kann vorteilhaft eine Rasteinrichtung vorgesehen sein, mit der der Fanghaken an dem Griffteil festgesetzt werden kann, an dem er schwenkbar gelagert ist, z. B. in der Stellung, in der der Fanghaken das gegenüberliegende Griffteil freigibt. Auf diese Weise kann bei einem häufig aufeinanderfolgenden Öffnen und Schließen des Werkzeuges der Fanghaken nicht versehentlich in seine Schließstellung geraten, das Werkzeug in seiner geschlossenen Stellung verriegeln und auf diese Weise die Arbeit behindern.

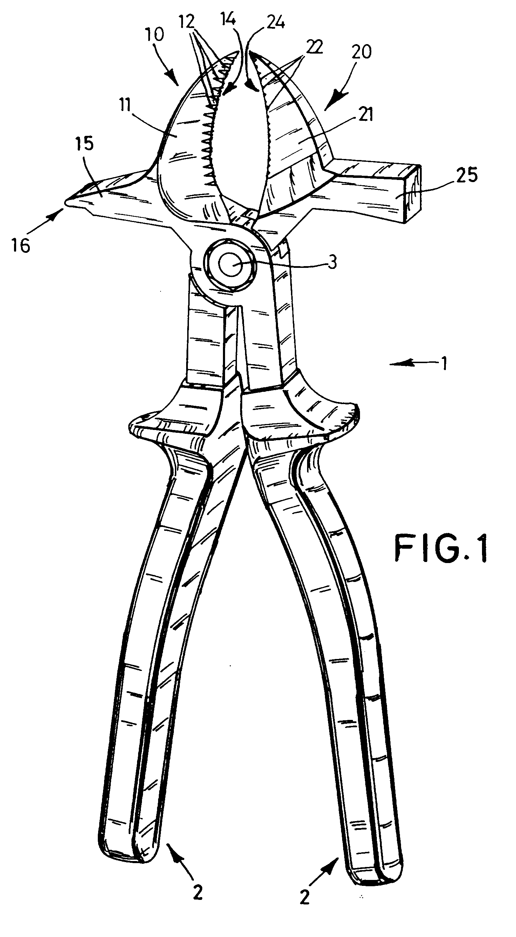

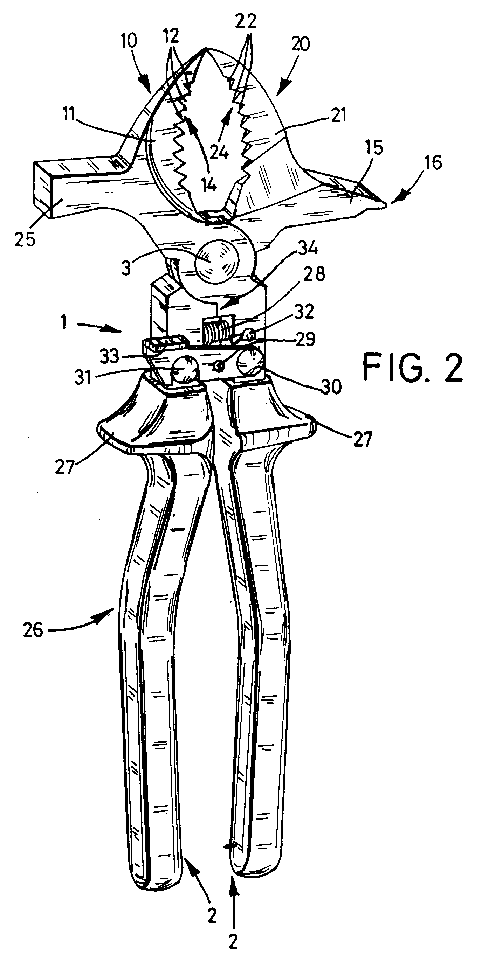

Die Erfindung wird anhand der Zeichnungen im folgenden näher erläutert. Dabei zeigen die
- Fig. 1 und 2: perspektivische Ansichten zweier zangenartiger Kombinations-Dentalwerkzeuge.

In Fig. 1 ist mit 1 allgemein ein Kombinations-Dentalwerkzeug bezeichnet, welches zwei Griffteile 2 umfaßt sowie ein Gelenk 3 und zwei Wirkteile 10 und 20.

Die beiden Wirkteile 10 und 20 umfassen zunächst zwei Backen 11 und 21, die zusammen eine Gipsschere bilden, wobei die Backen 11 und 21 Vertiefungen 12 und 22 aufweisen, die sich nutenartig in den Backenflächen bis zu den Schneidkanten 14 und 24 erstrecken und die dabei etwa senkrecht zur Längsachse des Kombinations-Dentalwerkzeuges 1 bzw. senkrecht zur Schneidkante 14 bzw. 24 verlaufen.

Weiterhin umfaßt das Kombinations-Dentalwerkzeug 1 an seinem Wirkteil 10 eine Hammerspitze 15, die an ihrem vorderen Ende eine schmale vordere Kante 16 aufweist. Diese vordere Kante 16 ist im Vergleich zu handelsüblichen Hämmern schmaler ausgebildet und gegenüber der übrigen Hammerspitze 15 stufenartig abgesetzt. Auf diese Weise wird einerseits durch die Masse der Hammerspitze die Schlagwirkung und die Stabilität des Hammeranteiles des Kombinationswerkzeuges verbessert und andererseits eine schmale Wirkkante an der Hammerspitze erzielt, mit der beispielsweise das Eindringen zwischen Gipsmodell und Sockelplatte aus Kunststoff verbessert und erleichtert wird.

Das Wirkteil 20 weist einen Hammerkopf 25 auf, wobei die Hammerspitze 15 und der Hammerkopf 25 im wesentlichen senkrecht zur Längsachse des Kombinations-Dentalwerkzeuges 1 und im wesentlichen achsgleich ausgerichtet sind.

Mit Hilfe der Backen 11 und 21 der Gipsschere können bei einem Gipsmodell eines Zahnabdruckes vorstehende untere Bereiche unterhalb des eigentlichen Abdruckes entfernt werden. Mit Hilfe der schmalen vorderen Kante 16 der Hammerspitze 15 kann durch gezielte Schläge auf die Trennfuge zwischen der Sockelplatte und dem eigentlichen Gipsmodell dieses von der Sockelplatte gelöst werden, ohne daß weitere zusätzliche Werkzeuge erforderlich sind.

Auf diese Weise dient insgesamt das Kombinations-Dentalwerkzeug 1 bei der Behandlung von Gipsmodellen vom Öffnen des metallischen Formkastens bis zur endgültigen Bearbeitung, ohne daß Werkzeugwechsel erforderlich werden, die das Arbeiten umständlich machen.

In Fig. 2 ist ein Kombinationswerkzeug ähnlich dem aus Fig. 1 dargestellt. Im Unterschied zum ersten Ausführungsbeispiel sind die Vertiefungen 12 und 22 in den beiden Schneidkanten 14 und 24 nicht als Nuten in der Schneidfläche ausgebildet, sondern als Vertiefungen in der inneren Kontur der Backen 11 und 21, so daß diese Konturen insgesamt gezahnt ausgebildet sind.

Durch diese zahnartige Kontur kann mit Hilfe des Werkzeuges 1 der Gips regelrecht geraspelt werden. Im Gegensatz zu widerhakenartig verlaufenden Vertiefungen stellt die Anordnung der Vertiefungen 12 und 22 sicher, daß sich selbst feuchter Gips nicht in ihnen festsetzen kann und daß die Raspelwirkung des Werkzeuges 1 stets erhalten bleibt.

Weiterhin ist an den Griffteilen 2 ein Griffbereich 26 angedeutet, wobei dieser Griffbereich vom freien Ende der Griffteile 2 bis etwa zu einem Handschutz 27 verläuft, der durch die Ummantelung der Griffteile gebildet ist.

Zwischen dem eigentlichen Griffbereich 26 und dem Gelenk 3 weist das in Fig. 2 dargestellte zweite Ausführungsbeispiel eine Feder 28 auf, welche die beiden Griffteile 2 spreizt und auf diese Weise das Öffnen des Werkzeuges 1 erleichtert, so daß bei einer Handhabung mit wiederholtem Öffnen und Schließen des Werkzeuges die Handhabung insgesamt erleichtert wird.

Weiterhin weist das Ausführungsbeispiel gemäß Fig. 2 einen Fanghaken 29 auf, der an dem rechten Griffteil 2 bei 30 schwenkbar gelagert ist und dem ein Vorsprung 31 an dem gegenüberliegenden Griffteil 2 zugeordnet ist. In der dargestellten geschlossenen Stellung des Kombinationswerkzeuges 1 umgreift der Fanghaken 29 den Vorsprung 31, wobei der Vorsprung 31 pilzartig ausgebildet ist und der Fanghaken 29 zwischen dem links dargestellten Griffteil 2 und dem Kopf des Vorsprunges 31 festgeklemmt und auf diese Weise festgelegt ist.

Alternativ zu einer derartigen Klemmung kann in Abwandlung des dargestellten Ausführungsbeispiels der Fanghaken 29 durch eine Feder in seiner Verriegelungsstellung gehalten werden, wobei die Feder unterhalb des Kopfes des Vorsprunges 31 angeordnet ist und eine unbeabsichtigte Entriegelung des Fanghakens verhindert.

Eine derartige Feder kann beispielsweise in einer Nut angeordnet sein, die in der Mantelfläche des Schaftes des pilzartigen Vorsprunges 31 vorgesehen ist. Die Feder kann aus einem Federstahldraht bestehen, der sich gegenüber der Mantelfläche des Schaftes konvex vorwölbt.

Der Fanghaken 29 ist durch eine Platte gebildet, die einen abgewinkelten Bereich 33 aufweist. Mit dem Daumen der das Werkzeug haltenden Hand kann daher der Fanghaken 29 betätigt werden, ohne umzugreifen oder das Werkzeug aus der Hand zu nehmen oder eine weitere Hand zu benutzen. Hierdurch wird die Handhabung des Werkzeuges erleichtert und eine höhere Arbeitsgeschwindigkeit mit dem Werkzeug ermöglicht.

Damit der Fanghaken 29 in seiner geöffneten Stellung, in der er das gegenüberliegende Griffteil 2 freigibt, nicht zu Behinderungen führt, weist der Fanghaken 29 an seiner zu den beiden Griffteilen 2 gerichteten Fläche einen Vorsprung oder eine Verdickung auf, wobei in dem Griffteil 2, an dem der Fanghaken 29 schwenkbar gelagert ist, eine Vertiefung 32 vorgesehen ist, die als Rasteinrichtung mit dem Vorsprung des Fanghakens 29 zusammenwirkt und den Fanghaken 29 in seiner geöffneten Stellung fixiert. Eine Abrundung oder eine Fase kann dabei an der Innenkante des rechten Griffteiles 2 vorgesehen sein, um das Verschwenken des Fanghakens 29 und insbesondere das Aufschieben seines Vorsprunges auf das rechte Griffteil 2 zu erleichtern, bis dieser Vorsprung in der Vertiefung 32 aufgenommen ist.

Unterhalb des Gelenkes 3 ist eine Kontaktzone 34 vorgesehen, an der die beiden Griffteile 2 aneinander anliegen. Die Übertragung der Schlagenergie bei geschlossenem Werkzeug wird hierdurch verbessert. Die Anordnung der Kontaktzone 34 unterhalb des Gelenkes 3, also im Abstand von den beiden Wirkteilen 10 und 20, verhindert das Eindringen von Gips oder ähnlichen Verschmutzungen, die das Schließen des Werkzeuges behindern könnten.

## Patentansprüche

1. Zangenartiges Kombinationswerkzeug (1) mit zwei Griffteilen (2), einem Gelenk (3) sowie zwei als Backen einer Zange ausgebildeten Wirkteilen (10, 20), wobei an einer ersten Backe (21) ein Hammerkopf (25) und an der zweiten Backe (11) eine Hammerspitze (15) angeformt ist, wobei der Hammerkopf und die Hammerspitze achsgleich ausgerichtet sind und im wesentlichen senkrecht zur Längsachse des Werkzeuges verlaufen, dadurch gekennzeichnet, daß die beiden Wirkteile (10, 20) als Backen (11, 21) einer Gipsschere sichelförmig gebogen ausgebildet sind.

2. Kombinationswerkzeug nach Anspruch 1, dadurch gekennzeichnet, daß an der Hammerspitze (15) eine schmale vordere Kante (16) stufenförmig angeformt ist.

3. Kombinationswerkzeug nach Anspruch 1 oder 2, dadurch gekennzeichnet, daß in den Schneidkanten (14, 24) der Gipsschere Vertiefungen (12, 22) angeordnet sind, die im wesentlichen senkrecht zu der Schneidkante (14, 24) verlaufen.

4. Kombinationswerkzeug nach Anspruch 3, dadurch gekennzeichnet, daß die Schneidkanten (14, 24) mittels der Vertiefungen (12, 22) gezahnt ausgebildet sind.

5. Kombinationswerkzeug nach einem der vorhergehenden Ansprüche, dadurch gekennzeichnet, daß zwischen den beiden Griffteilen (2) eine Feder angeordnet ist, welche die beiden Griffteile (2) spreizt, wobei die Feder zwischen dem Gelenk (3) und dem Griffbereich der Griffteile (2) angeordnet ist.

6. Kombinationswerkzeug nach einem der vorhergehenden Ansprüche, dadurch gekennzeichnet, daß zwischen dem Griffbereich und dem Gelenk (3) an einem der Griffteile (2) ein Fanghaken schwenkbar angeordnet ist, dem ein Vorsprung an dem anderen Griffteil (2) zugeordnet ist, um das Werkzeug (1) im geschlossenen Zustand zu halten.

7. Kombinationswerkzeug nach Anspruch 6, dadurch gekennzeichnet, daß dem Fanghaken an dem Griffteil (2), an dem er schwenkbar gelagert ist, eine Rasteinrichtung zugeordnet ist zur Festlegung des Fanghakens in seiner das andere Griffteil (2) freigebenden Stellung.

## Claims

1. Plier-like combination tool (1) having two grip parts (2), an articulation (3), and two active parts (10, 20) which are designed as plier jaws, a hammer head (25) being integrally formed on a first jaw (21) and a hammer tip (15) being integrally formed on the second jaw (11), the hammer head and the hammer tip being aligned equiaxially and running essentially perpendicularly with respect to the longitudinal axis of the tool, characterized in that the two active parts (10, 20) are designed to be curved in a sickle-like manner as jaws (11, 21) of plaster shears.

2. Combination tool according to Claim 1, characterized in that a narrow front edge (16) is integrally formed in a step-like manner on the hammer tip (15).

3. Combination tool according to Claim 1 or 2, characterized in that depressions (12, 22) are arranged in the cutting edges (14, 24) of the plaster shears and run essentially perpendicularly with respect to the cutting edge (14, 24).

4. Combination tool according to Claim 3, characterized in that the cutting edges (14, 24) are designed in a toothed manner by means of the depressions (12, 22).

5. Combination tool according to one of the preceding claims, characterized in that a spring is arranged between the two grip parts (2) and spreads apart the two grip parts (2), the spring being arranged between articulation (3) and the grip region of the grip parts (2).

6. Combination tool according to one of the preceding claims, characterized in that a catch hook is arranged pivotably, between the grip region and the articulation (3), on one of the grip parts (2) and is assigned a protrusion on the other grip part (2) in order to keep the tool (1) in the closed state.

7. Combination tool according to Claim 6, characterized in that a latching means is assigned to the catch hook, on the grip part (2) on which the latter is pivotably mounted, in order to secure the catch hook in its position in which it releases the other grip part (2).

## Revendications

1. Outil combiné du type pince (1) avec deux parties de préhension (2), une articulation (3) ainsi que deux parties actives (10, 20) formées comme des mâchoires d'une pince, une tête de marteau (25) sur une première mâchoire (21) et une pointe de marteau (15) sur la deuxième mâchoire (11) étant formées, la tête de marteau et la pointe de marteau étant centrées sur le même axe et s'étendant sensiblement perpendiculairement par rapport à l'axe longitudinal de l'outil, caractérisé en ce que les deux parties actives (10, 20) sont formées comme des mâchoires (11, 21) d'un ciseau à gypse courbées en forme de croissant.

2. Outil combiné selon la revendication 1, caractérisé en ce qu'une arête avant étroite (16) en gradins est formée sur la pointe de marteau (15).

3. Outil combiné selon la revendication 1 ou 2, caractérisé en ce que, dans les arêtes de coupe (14, 24) du ciseau à gypse, des creux (12, 22) sont agencés, qui s'étendent sensiblement perpendiculairement à l'arête de coupe (14, 24).

4. Outil combiné selon la revendication 3, caractérisé en ce que les angles de coupe (14, 24) sont dentelés au moyen des creux (12, 22).

5. Outil combiné selon l'une quelconque des revendications précédentes, caractérisé en ce qu'entre les deux parties de préhension (2) est agencé un ressort, lequel écarte les deux parties de préhension (2), le ressort étant agencé entre l'articulation (3) et la zone de préhension des parties de préhension (2).

6. Outil combiné selon l'une quelconque des revendications précédentes, caractérisé en ce qu'entre la zone de préhension et l'articulation (3), un crochet d'arrêt est monté de manière à pivoter sur l'une des parties de préhension (2), auquel correspond une partie en saillie sur l'autre partie de préhension (2), afin de maintenir l'outil (1) à l'état fermé.

7. Outil combiné selon la revendication 6, caractérisé en ce que sur le crochet d'arrêt sur la partie de préhension (2), sur laquelle il est logé de manière pivotante, un dispositif d'encliquetage est agencé pour la fixation du crochet d'arrêt dans sa position assurant la libération de l'autre partie de préhension (2).
